(19) Europäisches Patentamt
European Patent Office
Office européen des brevets

(11) **EP 3 520 752 B1**

(12) **EUROPEAN PATENT SPECIFICATION**

(45) Date of publication and mention
of the grant of the patent:
**12.08.2020 Bulletin 2020/33**

(21) Application number: **16917770.6**

(22) Date of filing: **30.09.2016**

(51) Int Cl.:
*A61F 13/15* *(2006.01)*          *A61F 13/47* *(2006.01)*
*A61F 13/533* *(2006.01)*

(86) International application number:
**PCT/JP2016/079189**

(87) International publication number:
**WO 2018/061220 (05.04.2018 Gazette 2018/14)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**07.08.2019 Bulletin 2019/32**

(73) Proprietor: **Unicharm Corporation
Ehime 799-0111 (JP)**

(72) Inventor: **KURODA, Kenichiro
Kanonji-shi
Kagawa 769-1602 (JP)**

(74) Representative: **Staeger & Sperling
Partnerschaftsgesellschaft mbB
Sonnenstraße 19
80331 München (DE)**

(56) References cited:
**JP-A- 2008 144 328     JP-A- 2010 531 195
JP-A- 2013 075 082     JP-A- 2013 075 082
JP-A- 2016 123 636     US-A1- 2009 012 491**

**Description**

FIELD

The present disclosure relates to an absorbent article.

BACKGROUND

Absorbent articles, such as sanitary napkins and disposable diapers are commonly provided with colors that are recognizable by the wearer from the skin contact surface sides of the absorbent articles, for the purpose of increasing their decorative properties.

For example, PTL 1 describes an absorbent article having a liquid-penneable front sheet, a liquid-impermeable back sheet and an absorbent body disposed between the front sheet and back sheet, the absorbent body comprising a colored layer and an absorbing layer situated further toward the front sheet side than the colored layer, wherein the absorbent body has compressed sections formed by compressing the front sheet, absorbing layer and colored layer from the front sheet side to the back sheet side, the colored layer has layered colored fibers, and the absorbing layer has fibers layered by an airlaid method, without a binder binding together the fibers.

[CITATION LIST]

[PATENT LITERATURE]

[PTL 1] Japanese Unexamined Patent Publication No. 2013-75082. Further prior art in this technical field is disclosed in documents US 2009/012491 A1 and JP 2013 075082 A.

SUMMARY

[TECHNICAL PROBLEM]

In the absorbent article described in PTL 1, the compressed sections are formed by compressing the front sheet, absorbing layer and colored layer, allowing the colored layer to be visible from the front sheet side through the compressed sections. However, investigation by the present inventors has shown that there is room for improvement in how the location of the colored layer is used as a mark for matching with the location where it is to be placed on the wearer.

It is therefore an object of the present disclosure to provide an absorbent article with which it is easy to discern the positioning place from the liquid-permeable sheet side, and it is easy to match the location where the absorbent article should be positioned when it is put on, with the location where it is to be placed on the wearer.

[SOLUTION TO PROBLEM]

The present invention is defined by the features of claim 1 and refers to an absorbent article comprising a liquid-permeable sheet, an absorbent body, a colored layer and a liquid-impermeable sheet, in that order, and having a lengthwise direction, a widthwise direction and a thickness direction, wherein the absorbent article comprises an embossed region including a pair of embossed sections and a non-embossed section situated between them, each of the pair of embossed sections has the absorbent body embossed but does not have the colored layer embossed, and the non-embossed section does not have the absorbent body and colored layer embossed, and in the embossed region, thicknesses of embossed section-forming portions of the absorbent body forming each of the pair of embossed sections are smaller than a thickness of a non-embossed section-forming portion of the absorbent body forming the non-embossed section, wherein in the embossed region, a basis weight of the embossed section-forming portions of the absorbent body is lower than a basis weight of the non-embossed section-forming portion of the absorbent body.

[ADVANTAGEOUS EFFECTS OF INVENTION]

With the absorbent article of the present disclosure, it is easy to discern the positioning place from the liquid-permeable sheet side, and it is easy to match the location where the absorbent article should be positioned when it is put on, with the location where it is to be placed on the wearer.

BRIEF DESCRIPTION OF DRAWINGS

FIG. 1 is a plan view of an absorbent article 1 according to a first embodiment.

FIG. 2 is a partial cross-sectional view of the absorbent article 1, on cross-section II-II of FIG. 1.

FIG. 3 is a cross-sectional view corresponding to cross-section 11-11 in FIG. 1 for the absorbent body 5 before formation of embossed sections 21 to embossed sections 25.

FIG. 4 is a diagram for illustration of an absorbent article 1 according to a second embodiment.

FIG. 5 is a plan view of an absorbent article 1 according to a third embodiment.

FIG. 6 is a partial cross-sectional view of the absorbent article 1, on cross-section VI-VI of FIG. 5.

FIG. 7 is a plan view of an absorbent article 1 according to a fourth embodiment.

FIG. 8 is a plan view of an absorbent body 5.

FIG. 9 is a partial cross-sectional view of the absorbent article 1, on cross-section IX-IX of FIG. 7.

FIG. 10 is a cross-sectional view corresponding to cross-section IX-IX in FIG. 7 for the absorbent body 5 before formation of embossed sections 21 (21a, 21b).

FIG. 11 is a diagram for illustration of a production example for the absorbent core 7 of the absorbent article 1 according to the first embodiment.

DESCRIPTION OF EMBODIMENTS

Specifically, the present disclosure relates to the following aspects.

[Aspect 1]

An absorbent article comprising a liquid-permeable sheet, an absorbent body, a colored layer and a liquid-impermeable sheet, in that order, and having a lengthwise direction, a widthwise direction and a thickness direction, wherein the absorbent article comprises an embossed region including a pair of embossed sections and a non-embossed section situated between them,

each of the pair of embossed sections has the absorbent body embossed but does not have the colored layer embossed,

the non-embossed section does not have the absorbent body and colored layer embossed, and

in the embossed region, thicknesses of embossed section-forming portions of the absorbent body forming each of the pair of embossed sections are smaller than a thickness of a non-embossed section-forming portion of the absorbent body which forms the non-embossed section.

The absorbent article has, in the embossed region, prescribed embossed sections and a prescribed non-embossed section, the thicknesses of the embossed section-forming portions of the absorbent body being smaller than the thickness of the non-embossed section-forming portion of the absorbent body. In the embossed region of this absorbent article, therefore, the physical distance from the skin-contacting surface of the liquid-permeable sheet up to the colored layer in the embossed sections is closer than in the non-embossed section, and therefore the colored layer is easily visible through the embossed sections while the colored layer is less visible through the non-embossed section. Thus, when the wearer puts on the absorbent article, or a user fits the absorbent article onto the wearer, it is easy to match the location of the embossed region of the absorbent article with the location where it is to be placed on the wearer (for example, the vaginal opening, urine orifice or anus).

Throughout the present specification, cases in which the wearer puts on the absorbent article (for example, a sanitary napkin, panty liner or urine-absorbing pad) and cases where a user fits the absorbent article onto the wearer (for example, a disposable diaper or urine-absorbing pad) will be collectively expressed as the "wearer puts on the absorbent article".

Also throughout the present specification, matching the location of the embossed region of the absorbent article with the location where it is to be placed on the wearer (for example, the vaginal opening, urine orifice or anus) will be referred to simply as "positioning".

[Aspect 2]

The absorbent article according to aspect 1, wherein in the embossed region, a basis weight of the embossed section-forming portions of the absorbent body is lower than a basis weight of the non-embossed section-forming portion of the absorbent body.

Since the basis weight of the embossed section-forming portions of the absorbent body is lower than the basis weight of the non-embossed section-forming portion of the absorbent body in the absorbent article, incident light directed onto the embossed sections from the liquid-permeable sheet side and reflected light that has been reflected by the colored layer and has returned to the liquid-permeable sheet side are unlikely to be attenuated, and the color of the embossed sections are easily visible to the wearer. At the non-embossed section, on the other hand, incident light that is directed to the non-embossed section from the liquid-permeable sheet side and reflected light that has been reflected by the colored layer and returned to the liquid-permeable sheet side, are more easily attenuated than at the embossed sections. As a result, the embossed region of the absorbent article is easily visible to the wearer, and positioning is facilitated when the absorbent article is put on.

[Aspect 3]
The absorbent article according to aspect 1 or 2, wherein the absorbent body comprises an absorbent core including pulp fibers and a polymer absorbent, and in the absorbent core, a mass ratio of the pulp fibers of the embossed section-forming portions of the absorbent core forming each of the pair of embossed sections is higher than a mass ratio of the pulp fibers of the non-embossed section-forming portion of the absorbent core forming the non-embossed section.

In this absorbent article, since the mass ratio of pulp fibers of the embossed section-forming portions of the absorbent core is higher than the mass ratio of pulp fibers of the non-embossed section-forming portion of the absorbent core, incident light directed onto the embossed sections from the liquid-permeable sheet side and reflected light that has been reflected by the colored layer and returned to the liquid-permeable sheet side, are unlikely to be reflected at the interface between the pulp fibers and the polymer absorbent that has a different refractive index than the pulp fibers, and the color of the embossed sections is easily visible to the wearer. At the non-embossed section, on the other hand, incident light that is directed to the non-embossed section from the liquid-permeable sheet side and reflected light that has been reflected by the colored layer and returned to the liquid-permeable sheet side, are easily reflected at the interface between the pulp fibers and the polymer absorbent, and the color of the non-embossed section is less visible to the wearer. As a result, the embossed region of the absorbent article is easily visible to the wearer, and positioning is facilitated when the absorbent article is put on.

[Aspect 4]
The absorbent article according to any one of aspects 1 to 3, wherein each of the pair of embossed sections has the liquid-permeable sheet further embossed in the thickness direction.
In this absorbent article, each of the embossed sections has the liquid-permeable sheet embossed, and therefore the liquid-permeable sheet in the embossed sections has more excellent light permeability than the liquid-permeable sheet in the non-embossed section. As a result, the embossed region of the absorbent article is easily visible to the wearer, and positioning is facilitated when the absorbent article is put on.

[Aspect 5]
The absorbent article according to any one of aspects 1 to 4, wherein the embossed region is disposed in a region including the excretory opening contact region of the absorbent article.
In this absorbent article, since the embossed region is disposed in a region including the excretory opening contact region, the wearer can easily position the absorbent article so that the location of the excretory opening of the wearer matches the location of a excretory opening contact region of the absorbent article.

[Aspect 6]
The absorbent article according to aspect 5, wherein the absorbent article has an anchoring part in the liquid-impermeable sheet for anchoring of the absorbent article to clothing, and the pair of embossed sections extend in the lengthwise direction at both outer sides of the non-embossed section in the widthwise direction.
Since the absorbent article is a type that is to be used by anchoring onto clothing, such as a sanitary napkin, panty liner or mine-absorbing pad, and the pair of embossed sections extend in the lengthwise direction on both outer sides of the non-embossed section in the widthwise direction, the embossed sections of the embossed region serve as folding origins in the widthwise direction of the absorbent article, and the absorbent article has excellent fittability. Furthermore, since the embossed sections of the embossed region allow flow of body fluid along the embossed sections in the lengthwise direction, the absorbent article has excellent leakage resistance.

[Aspect 7]
The absorbent article according to aspect 6, wherein the non-embossed section is disposed in the excretory opening contact region, and the pair of embossed sections continuously or intermittently surround an outer edge of the excretory opening contact region.
In this absorbent article, since the non-embossed section is disposed in the excretory opening contact region, and the pair of embossed sections continuously or intermittently surround the excretory opening contact region, the embossed region of the absorbent article is easily visible to the wearer, and positioning is facilitated when the absorbent article is put on. Moreover, since the embossed sections of the embossed region inhibit migration of body fluid to the outer sides, the absorbent article has excellent leakage resistance.

[Aspect 8]
The absorbent article according to aspect 7, wherein the absorbent article comprises, on an inner side of the embossed region, a second embossed region that includes a pair of second embossed sections and a second non-embossed section situated between them, each of the pair of second embossed sections has the liquid-permeable sheet and absorbent body embossed but does not have the colored layer embossed, and the second non-embossed section does not have the absorbent body and colored layer embossed.
Since the absorbent article further comprises the second embossed region on the inner side of the embossed region, the embossed region and second embossed region of the absorbent article are easily visible to the wearer, and positioning is facilitated when the absorbent article is put on.

[Aspect 9]
The absorbent article according to any one of aspects 1 to 5, wherein the absorbent article is a disposable diaper, and each of the pair of embossed sections is a folding guide extending in the lengthwise direction.
Since the absorbent article is a disposable diaper and the embossed sections are folding guides extending in the lengthwise direction, the absorbent article has excellent fittability.

[Aspect 10]
The absorbent article according to any one of aspects 1 to 9, wherein the absorbent article comprises a second absorbent body between the absorbent body and the liquid-impermeable sheet, with the colored layer forming a skin surface of the second absorbent body.
Since the absorbent article further includes a second absorbent body constituting the colored layer, a color shade may be selected that easily serves as a mark for positioning of the absorbent article from the liquid-permeable sheet side of the absorbent article, regardless of the color shade formed on the clothing-contacting surface of the liquid-impermeable sheet.

[Aspect 11]
The absorbent article according to any one of aspects 1 to 10, wherein the colored layer further includes a functional component.

Since the colored layer in this absorbent article further includes a functional component, the wearer associates the function of the functional component and the color of the colored layer, and the function of the functional component is more easily perceived.
The absorbent article of this disclosure will now be explained in detail.

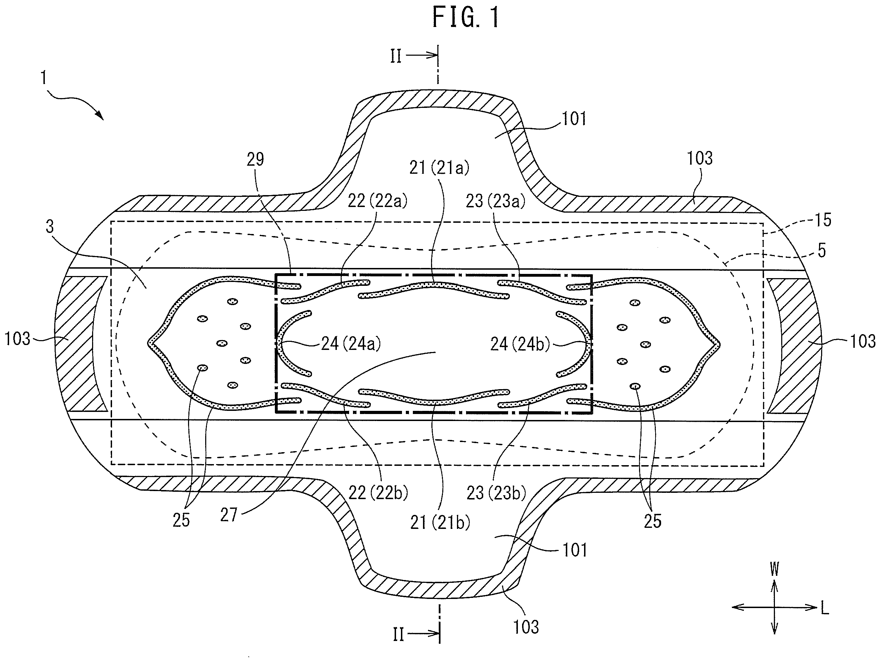

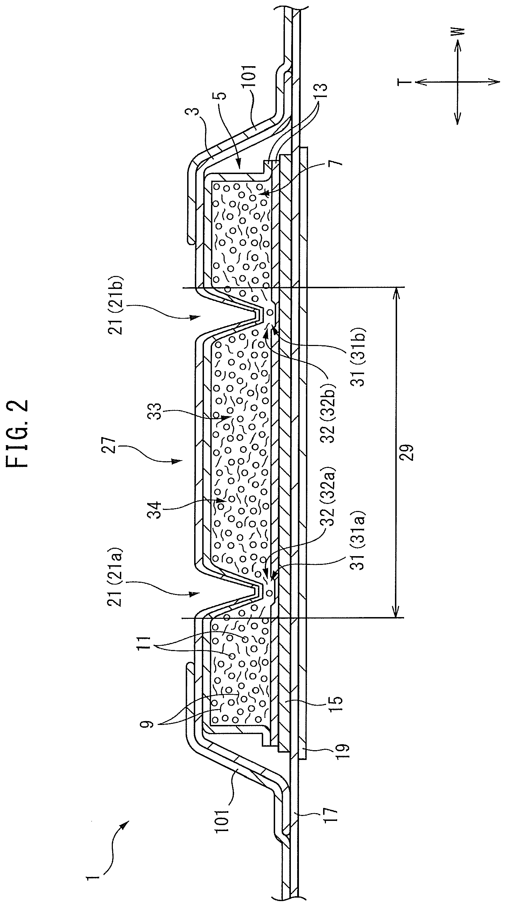

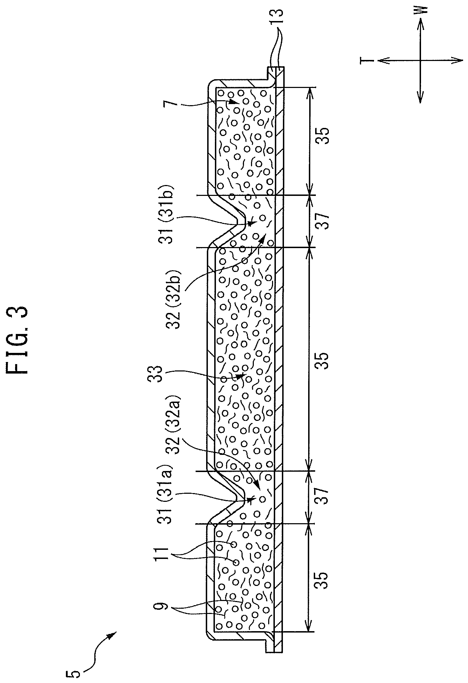

Fig. 1 to Fig. 3 are diagrams for illustration of absorbent article 1 according to one embodiment of the present disclosure (hereunder referred to as the "first embodiment"), which is specifically a sanitary napkin. Specifically, Fig. 1 is a plan view of the absorbent article 1. Fig. 2 is a partial cross-sectional view of the absorbent article 1, on cross-section II-II of (Fig. 1. Fig. 3 is a cross-sectional view corresponding to cross-section II-II in Fig. 1 for the absorbent body 5 before formation of embossed sections 21 to embossed sections 25.
The absorbent article 1 of the first embodiment comprises a liquid-permeable sheet 3, an absorbent body 5, a colored layer 15, a liquid-impermeable sheet 17, and an anchoring part 19 for anchoring of the absorbent article 1 to clothing. The absorbent article 1 also has a lengthwise direction L, a widthwise direction W and a thickness direction T. The absorbent article 1 has a side sheets 101 and seal sections 103 that seal the ends of the absorbent article 1, but these are all publicly known in the technical field and will not be explained in detail here.
The colored layer 15 has a longer length in the lengthwise direction L and a longer length in the widthwise direction W than the absorbent body 5, and it is disposed so that its edges are further outward than the edges of the absorbent body 5 in both the lengthwise direction L and the widthwise direction W. Also, the colored layer 15 is composed of a nonwoven fabric that is colored green, differing from the white liquid-permeable sheet.
The absorbent body 5 is composed of an absorbent core 7 and two core wraps 13 that cover the absorbent core 7, the absorbent core 7 including pulp fibers 9 and a polymer absorbent 11.
The absorbent article 1 comprises a pair of embossed sections 21 (21a, 21b), a pair of embossed sections 22 (22a, 22b), a pair of embossed sections 23 (23a, 23b), a pair of embossed sections 24 (24a, 24b) and embossed sections 25.
The absorbent article 1 comprises an embossed region 29 that includes the pair of embossed sections 21 (21a, 21b) disposed in a mutually separated manner in the widthwise direction W, and a non-embossed section 27 between them. The non-embossed section 27 is disposed at the center in the lengthwise direction L and widthwise direction W of the absorbent article 1, and in the excretory opening contact region of the absorbent article 1.

The pair of embossed sections 21 (21a, 21b) are situated on both outer sides of the non-embossed section 27 in the widthwise direction W, each of the pair of embossed sections 21 (21a, 21b) extending in the lengthwise direction L.

As shown in Fig. 2, each of the pair of embossed sections 21 (21a, 21b) have the liquid-permeable sheet 3 and absorbent body 5 embossed in the thickness direction T, while the colored layer 15 is not embossed. In the non-embossed section 27, none of the liquid-permeable sheet 3, absorbent body 5 and colored layer 15 are embossed.

In the embossed region 29, the basis weights of each of the pair of embossed section-forming portions 31 (31a, 31b), which are the portions of the absorbent body 5 forming the pair of embossed sections 21 (21a, 21b), are lower than the basis weight of the non-embossed section-forming portion 33, which is the portion of the absorbent body 5 forming the non-embossed section 27. As shown in Fig. 3, this is because at the point before the pair of embossed sections 21 (21a, 21b) are formed, the absorbent body 5, and specifically the absorbent core 7, comprises high-basis-weight regions 35 and low-basis-weight regions 37 of lower basis weight than the basis weight of the high-basis-weight regions 35, the low-basis-weight regions 37 being formed so as to match each of the pair of embossed section-forming portions 31 (31a, 31b). The method for producing the absorbent core 7 is as follows.

Specifically, at the point before the pair of embossed sections 21 (21a, 21b) are formed, the pair of embossed section-forming portions 31 (31a, 31b) are the respective low-basis-weight regions 37 and the non-embossed section-forming portion 33 constitutes the high-basis-weight regions 35, so that even after the pair of embossed sections 21 (21a, 21b) have been formed, it still has the same basis weight relationship. When the color of the colored layer 15 is observed through the absorbent body 5, a lower basis weight of the absorbent body 5 allows the color of the colored layer 15 to penetrate more easily, and therefore from the liquid-permeable sheet 3 side, the color of the colored layer 15 is more easily visible through each of the pair of embossed sections 21 (21a, 21b), while the color of the colored layer 15 is less easily visible through the non-embossed section 27.

Furthermore, at the absorbent core 7 of the embossed region 29, the mass ratio of pulp fibers in each of the embossed section-forming portions of the absorbent core 32 (32a, 32b) composing the pair of embossed sections 21 (21a, 21b) is higher than the mass ratio of the pulp fibers 9 of the non-embossed section-forming portion of the absorbent core 34 composing the non-embossed section 27. As shown in Fig. 3, this is because at the point before the pair of embossed sections 21 (21a, 21b) are formed, the absorbent body 5, and specifically the absorbent core 7, has the mass ratio of the pulp fibers 9 in the low-basis-weight regions 37 corresponding to the pair of embossed section-forming portions 31 (31a, 31b), higher than the mass ratio of the pulp fibers 9 in the high-basis-weight regions 35 corresponding to the non-embossed section-forming portion, so that it has the same pulp proportion relationship even after the pair of embossed sections 21 (21a, 21b) have been formed.

In the absorbent core 7, it will be common for the refractive index of the pulp fibers 9 and the refractive index of the polymer absorbent 11 to be different, and for each of the pair of embossed sections 21 (21a, 21b) in the form of a film, a higher mass ratio of the pulp fibers 9 will cause incident light directed to each of the embossed sections 21 (21a, 21b) to be less likely to be reflected at the interface between the pulp fibers 9 and the polymer absorbent 11, more easily reaching the colored layer 15, compared to when the mass ratio of the pulp fibers 9 is low, and the incident light will reach the colored layer 15, and subsequently reflected light will more easily penetrate the embossed sections 21 (21a, 21b) and reach the eyes of the wearer.

At the non-embossed section 27, the absorbent core 7 includes pulp fibers 9 and the polymer absorbent 11 in a state that also includes air. In this condition, if the mass ratio of the pulp fibers 9 of the absorbent core 7 is lowered, i.e. if the mass ratio of the polymer absorbent 11 of the absorbent core 7 is increased, in the non-embossed section 27, then compared to when it is not, a greater amount of light will be reflected at the interface between the pulp fibers 9 and the polymer absorbent 11, the interface between the pulp fibers 9 and air, and the interface between the polymer absorbent 11 and air, while incident light directed onto the non-embossed section 27 will be unlikely to reach the colored layer 15, and reflected light that has been reflected from the colored layer 15 will be unlikely to pass through the non-embossed section 27 and will be unlikely to reach the eyes of the wearer.

By thus making the mass ratio of the pulp fibers 9 in the embossed section-forming portions of the absorbent core 32 (32a, 32b) higher than the mass ratio of the pulp fibers 9 in the non-embossed section-forming portion of the absorbent core 34, in the embossed region 29, the color of the colored layer 15 is more visible through the pair of embossed sections 21 (21a, 21b) while the color of the colored layer 15 is less visible through the non-embossed section 27, from the liquid-permeable sheet 3 side.

In addition, in the embossed region 29, the thickness of the embossed section-forming portions 31 (31a, 31b) of the absorbent body 5 is smaller than the thickness of the non-embossed section-forming portion 33 of the absorbent body 5. Thus, at the pair of embossed sections 21 (21a, 21b), the distance between the skin-contacting surface of the liquid-permeable sheet 3 and the colored layer 15 is physically closer than that in the non-embossed section 27, and the color of the colored layer 15 is more easily visible through the pair of embossed sections 21 (21a, 21b) while the color of the colored layer 15 is less easily visible through the non-embossed section 27, from the liquid-permeable sheet 3 side.

In particular, in the absorbent body 5 at the point before the pair of embossed sections 21 (21a, 21b) are formed, the thicknesses of the embossed section-forming portions 31 (31a, 31b) forming the pair of embossed sections 21 (21a, 21b) are smaller than the thickness of the non-embossed section-forming portion 33 forming the non-embossed section 27. Specifically, in the absorbent core 7, at the point before the pair of embossed sections 21 (21a, 21b) are formed, the thicknesses of the embossed section-forming portions of the absorbent core 32 (32a, 32b) forming the pair of embossed sections 21 (21a, 21b) are smaller than the thickness of the non-embossed section-forming portion of the absorbent core 34 forming the non-embossed section 27.

Therefore, at the pair of embossed sections 21 (21a, 21b), the distance between the skin-contacting surface of the liquid-permeable sheet 3 and the colored layer 15 is physically closer than in the non-embossed section 27, and the color of the colored layer 15 is more easily visible through the pair of embossed sections 21 (21a, 21b) while the color of the colored layer 15 is less easily visible through the non-embossed section 27, from the liquid-permeable sheet 3 side.

Similarly, the embossed region 29 includes the pair of embossed sections 22 (22a, 22b) disposed in a mutually separated manner in the widthwise direction W, and a non-embossed section 27 between them, while also including the pair of embossed sections 23 (23a, 23b) disposed in a mutually separated manner in the widthwise direction W and a non-embossed section 27 between them, and including the pair of embossed sections 24 (24a, 24b) disposed in a mutually separated manner in the lengthwise direction L and a non-embossed section 27 between them, but since the relationship is the same as between the pair of embossed sections 21 (21a, 21b) disposed in a mutually separated manner in the widthwise direction W and the non-embossed section 27 between them, it will not be explained again.

In other words, for the first embodiment, the embossed region 29 has the non-embossed section 27 disposed in the excretory opening contact region and the embossed sections (21, 22, 23, 24) disposed in a manner intermittently surrounding the non-embossed section 27. This makes the embossed region 29 of the absorbent article 1 more easily visible to the wearer, and facilitates matching of the location of the embossed region 29 of the absorbent article 1 with the location where it is to be placed on the wearer, when the absorbent article 1 is put on. Moreover, since the embossed sections (21, 22, 23, 24) of the embossed region 29 inhibit migration of body fluid to the outer sides, the absorbent article has excellent leakage resistance.

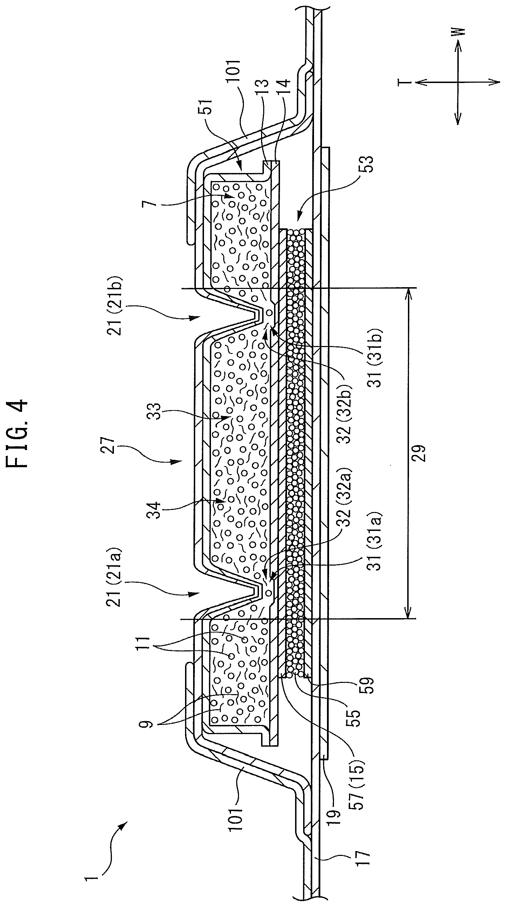

Fig. 4 is a diagram for illustration of absorbent article 1 according to another embodiment of the present disclosure (hereunder referred to as the "second embodiment"), which is specifically a sanitary napkin. Fig. 4 is a cross-sectional view corresponding to cross-section II-II of Fig. 1.

The absorbent article 1 of the second embodiment comprises a liquid-permeable sheet 3, a first absorbent body 51, a second absorbent body 53, a liquid-impermeable sheet 17, and an anchoring part 19 for anchoring of the absorbent article 1 to clothing, in that order. The first absorbent body 51 comprises an absorbent core 7, a core wrap 13 covering the absorbent core 7 from the liquid-permeable sheet 3 side, and a core wrap 14 covering the absorbent core 7 from the liquid-impermeable sheet 17 side, the absorbent core 7 including pulp fibers 9 and a polymer absorbent 11. In the first absorbent body 51, the core wrap 13 covering the absorbent core 7 from the liquid-impermeable sheet 17 side is colored green.

The second absorbent body 53 comprises a second upper core wrap 57 next to the liquid-permeable sheet 3, a second lower core wrap 59 next to the liquid-impermeable sheet 17, and a polymer absorbent layer 55 between the second upper core wrap 57 and second lower core wrap 59. The second upper core wrap 57 forms the skin side surface of the second absorbent body 53. The second upper core wrap 57 is colored with a green color having higher chroma than the core wrap 13 of the first absorbent body 51, and it constitutes the colored layer 15.

Since the absorbent article 1 according to the second embodiment comprises the first absorbent body 51 and second absorbent body 53, wherein the second absorbent body 53 constitutes the colored layer 15, a color shade may be selected that serves as a mark for positioning of the absorbent article 1 from the liquid-permeable sheet 3 side of the absorbent article 1, regardless of the color shade formed on the clothing-contacting surface of the liquid-impermeable sheet.

Also, since the absorbent article according to the second embodiment has the core wrap 13 of the first absorbent body 51 colored in addition to the colored layer 15, and specifically since the colored layer 15 and the core wrap 13 have green colors, with the chroma of the colored layer 15 being higher than the chroma of the core wrap 13, the color of the embossed sections 21 appears vivid in the embossed region 29. Therefore, the embossed region 29 of the absorbent article 1 is easily visible to the wearer, and facilitates matching of the location of the embossed region 29 of the absorbent article 1 with the location where it is to be placed on the wearer, when the absorbent article 1 is put on. Furthermore, since positioning of the absorbent article 1 is easy, it can be more easily fitted in the proper manner, and as a result, leakage is less likely to occur in the absorbent article 1.

The other portions are the same as the absorbent article 1 of the first embodiment and will not be explained again here.

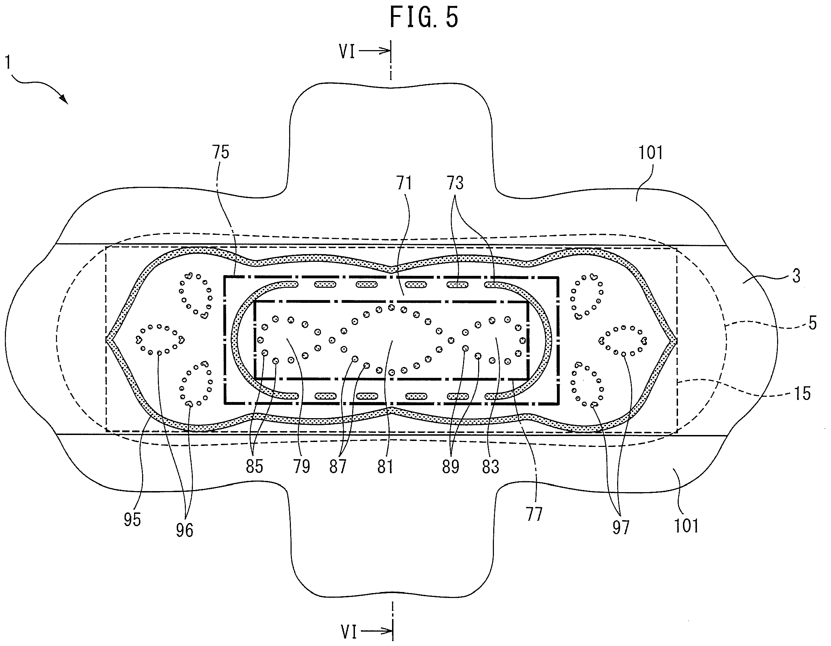

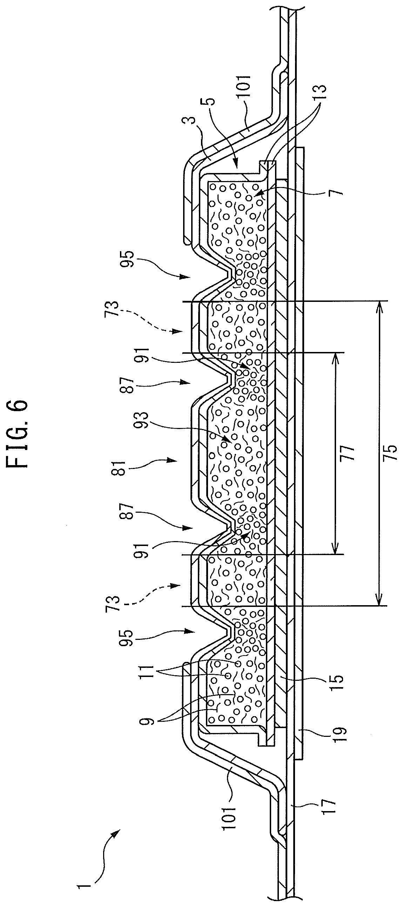

Fig. 5 and Fig. 6 are diagrams for illustration of absorbent article 1 according to another embodiment of the present disclosure (hereunder referred to as the "third embodiment"), which is specifically a sanitary napkin. Specifically, Fig. 5 is a plan view of the absorbent article 1. Fig. 6 is a partial cross-sectional view of the absorbent article 1, on cross-section VI-VI of Fig. 5.

The absorbent article 1 of the third embodiment comprises a liquid-permeable sheet 3, an absorbent body 5, a colored layer 15, a liquid-impermeable sheet 17, and an anchoring part 19 for anchoring of the absorbent article 1 to clothing, in that order. The absorbent article 1 also has a lengthwise direction L, a widthwise direction W and a thickness direction T.

Incidentally, the absorbent article 1 comprises side sheets 101, and embossed sections 95, embossed sections 96 and embossed sections 97 where the liquid-permeable sheet 3 and absorbent body 5 are embossed.

The colored layer 15 is composed of a nonwoven fabric that is colored green, differing from the white liquid-permeable sheet 3.

The absorbent body 5 is composed of an absorbent core 7 and two core wraps 13 that cover the absorbent core 7, the absorbent core 7 including pulp fibers 9 and a polymer absorbent 11.

The absorbent article 1 comprises a first embossed region 75 that includes a first non-embossed section 71 situated in the excretory opening contact region, and first embossed sections 73 intermittently surrounding the outer edges of the excretory opening contact region. The absorbent article 1 also comprises a second embossed region 77 situated inside the excretory opening contact region, and specifically in the first non-embossed section 71. The second embossed region 77 includes a second non-embossed section 79, second non-embossed section 81 and second non-embossed section 83, as three non-embossed sections adjacent in the lengthwise direction L, second embossed sections 85 intermittently surrounding the second non-embossed section 79, second embossed sections 87 intermittently surrounding the second non-embossed section 81, and second embossed sections 89 intermittently surrounding the second non-embossed section 83.

Specifically, in the first embossed sections 73 of the first embossed region 75, the liquid-permeable sheet 3 and absorbent body 5 are embossed in the thickness direction T, while the colored layer 15 is not embossed. Also, in the first non-embossed section 71 of the first embossed region 75, none of the liquid-permeable sheet 3, absorbent body 5 and colored layer 15 are embossed, except for the second embossed region 77 (specifically, the second embossed sections 85, second embossed sections 87 and second embossed sections 89).

In the second embossed sections 85, second embossed sections 87 and second embossed sections 89 of the second embossed region 77, the liquid-permeable sheet 3 and absorbent body 5 are embossed in the thickness direction T, while the colored layer 15 is not embossed. In the second non-embossed section 79, second non-embossed section 81 and second non-embossed section 83 of the second embossed region 77, none of the liquid-permeable sheet 3, absorbent body 5 and colored layer 15 are embossed.

In the first embossed region 75, the thickness of the first embossed sections 73 is smaller than the thickness of the first non-embossed section 71. Thus, at the first embossed sections 73, the distance between the skin-contacting surface of the liquid-permeable sheet 3 and the colored layer 15 is physically closer than that in the first non-embossed section 71, and the color of the colored layer 15 is more easily visible through the first embossed sections 73 while the color of the colored layer 15 is less easily visible through the first non-embossed section 71, from the liquid-permeable sheet 3 side.

In the second embossed region 77, the thicknesses of each of the second embossed sections 85, 87, 89 are smaller than the thicknesses of the respective second non-embossed sections 79, 81, 83. Thus, at each of the second embossed sections 85, 87, 89, the distance between the skin-contacting surface of the liquid-permeable sheet 3 and the colored layer 15 is physically closer than the distance in each of the second non-embossed sections 79, 81, 83, and the color of the colored layer 15 is more easily visible through the second embossed sections 85, 87, 89 while the color of the colored layer 15 is less easily visible through the second non-embossed sections 79, 81, 83, from the liquid-permeable sheet 3 side.

In the absorbent article 1 of the third embodiment, at the point before the first embossed region 75 and second embossed region 77 are formed, the absorbent body 5, and specifically the absorbent core 7, has approximately constant basis weight in the planar direction, and even after the first embossed region 75 and second embossed region 77 have been formed, the absorbent core 7 maintains approximately constant basis weight in the planar direction. In the first embossed region 75, therefore, the basis weight of the first embossed sections 73 is approximately equal to the basis weight of the first non-embossed section 71. Similarly, in the second embossed region 77, the basis weight of each of the second embossed sections 85, 87, 89 is approximately equal to the basis weight of each of the second non-embossed sections 79, 81, 83.

Moreover, in the absorbent article 1 of the third embodiment, at the point before the first embossed region 75 and second embossed region 77 are formed, the absorbent core 7 has an approximately constant mass ratio of the pulp fibers 9 in the planar direction, and even after the first embossed region 75 and second embossed region 77 have been formed, the absorbent core 7 maintains an approximately constant mass ratio of the pulp fibers 9 in the planar direction. In the first embossed region 75, therefore, the mass ratio of pulp fibers in the absorbent core first embossed section-forming portions (not shown) constituting the first embossed sections 73 of the absorbent core 7, is approximately equal to the mass ratio of pulp fibers in the absorbent core first non-embossed section-forming portion (not shown), constituting the first non-embossed section 71 of the absorbent core 7. Similarly, in the second embossed region 77, the mass ratio of the pulp fibers 9 in the absorbent core second embossed section-forming portions 91 constituting the second embossed sections 85, 87, 89 of the absorbent core 7, is approximately equal to the mass ratio of the pulp fibers 9 in the absorbent core second non-embossed section-forming portions 93 constituting each of the second non-embossed sections 79, 81, 83 of the absorbent core 7.

Since the absorbent article 1 of the third embodiment further includes the second embossed region 77 on the inner side of the first embossed region 75 situated in the excretory opening contact region, the locations of the first embossed region 75 and second embossed region 77 of the absorbent article 1 can be easily matched with the location where it is to be placed on the wearer, when the absorbent article 1 is put on.

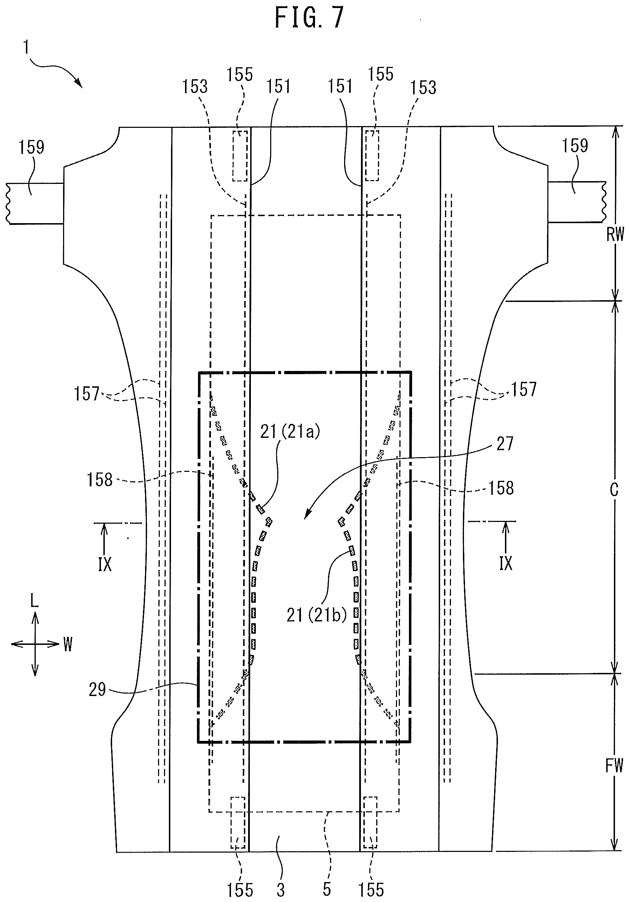

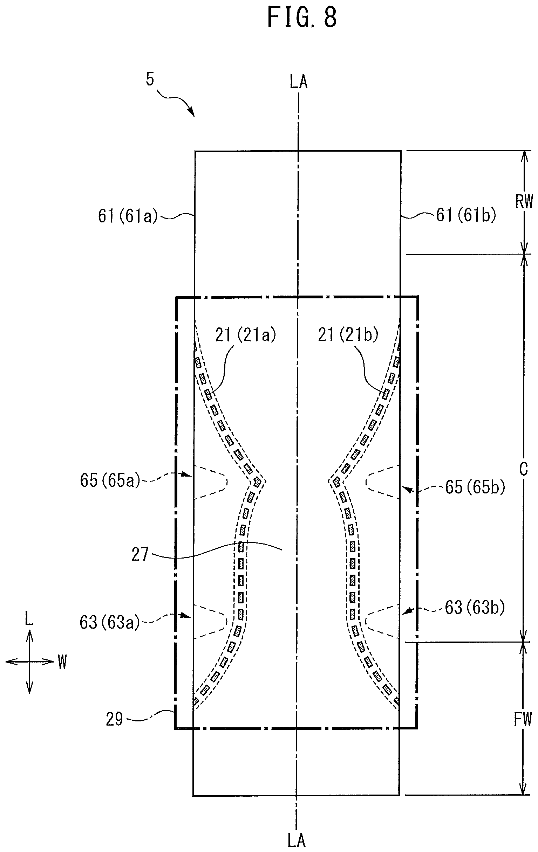

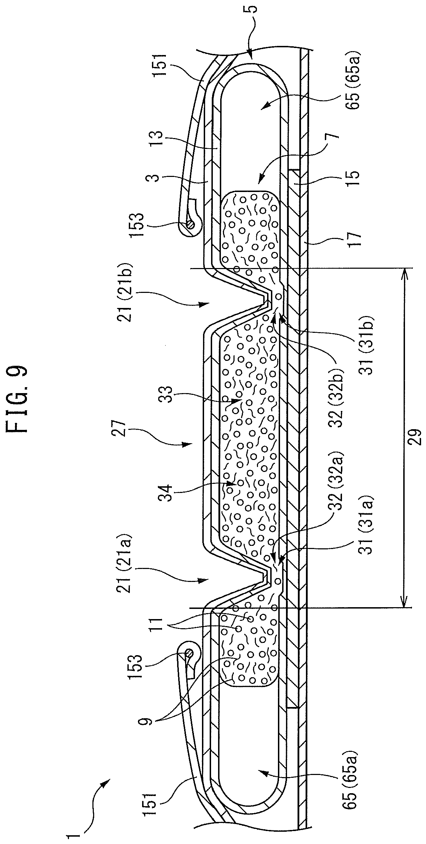

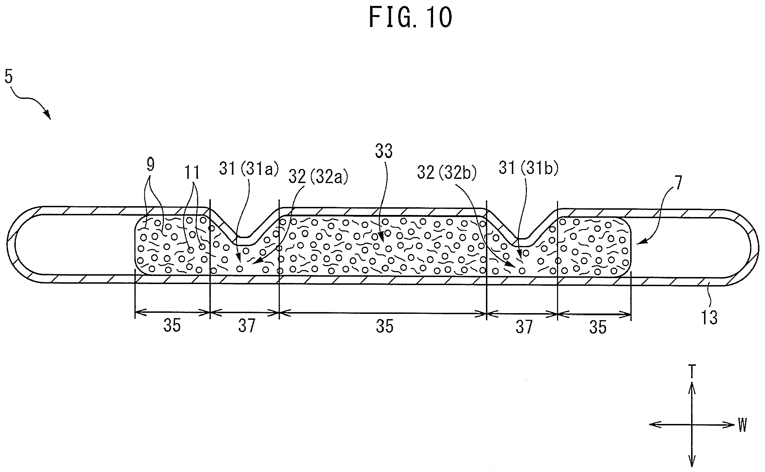

Fig. 7 to Fig. 10 are diagrams for illustration of absorbent article 1 according to yet another embodiment of the present disclosure (hereunder referred to as the "fourth embodiment"), which is specifically a disposable diaper. Specifically, Fig. 7 is a plan view of the absorbent article 1. Fig. 8 is a plan view of an absorbent body 5. Fig. 9 is a partial cross-sectional view of the absorbent article 1, on cross-section IX-IX of Fig. 7. Fig. 10 is a cross-sectional view corresponding to cross-section IX-IX in Fig. 7 for the absorbent body 5 before formation of embossed sections 21 (21a, 21b).

The absorbent article 1 of the fourth embodiment comprises a liquid-permeable sheet 3, an absorbent body 5, a colored layer 15 and a liquid-impermeable sheet 17, in that order. The colored layer 15 is composed of a resin film that is colored blue. The absorbent article 1 also has a lengthwise direction L, a widthwise direction W and a thickness direction T. The absorbent article 1 is partitioned into three regions in the lengthwise direction L: a front waist region FW, a rear waist region RW and a crotch region C between the front waist region FW and rear waist region RW.

The absorbent body 5 is composed of an absorbent core 7 and two core wraps 13 that cover the absorbent core 7, the absorbent core 7 including pulp fibers 9 and a polymer absorbent 11.

The absorbent article 1 has a pair of anti-leakage walls 151 including an elastic member 153, an anchoring part 155 for anchoring of the anti-leakage walls 151 to the liquid-permeable sheet 3, an elastic member 157, an elastic member 158 and a tape fastener 159, but because these are known in the technical field they will not be explained here.

The absorbent article 1 comprises an embossed region 29 that includes the pair of embossed sections 21 (21a, 21b) disposed in a mutually separated manner in the widthwise direction W, and a non-embossed section 27 between them. As shown in Fig. 8, each of the pair of embossed sections 21 (21a, 21b) extends intermittently in the lengthwise direction L across the front waist region FW and the crotch region C. The pair of embossed sections 21 (21a, 21b) also protrude toward the lengthwise axis line LA from each of the side edges 61 (61a, 61b) of the absorbent body 5 in the lengthwise direction L.

Each of the pair of embossed sections 21 (21a, 21b) functions as folding guides, since they are thinner and of lower basis weight than the non-embossed section 27. Therefore, when it is put on, the pair of embossed sections 21 (21a, 21b) easily folds the absorbent body 5, together with the pair of notch sections 63 (63a, 63b) and the pair of notch sections 65 (65a, 65b) formed in the absorbent core 7, with the pair of embossed sections 21 (21a, 21b) as origins, and hence the absorbent article 1 can fit onto the body of the wearer.

As shown in Fig. 9, each of the pair of embossed sections 21 (21a, 21b) have the liquid-permeable sheet 3 and absorbent body 5 embossed in the thickness direction T, while the colored layer 15 is not embossed. In the non-embossed section 27, none of the liquid-permeable sheet 3, absorbent body 5 and colored layer 15 are embossed.

In the embossed region 29, the basis weights of each of the pair of embossed section-forming portions 31 (31a, 31b) of the absorbent body 5 are lower than the basis weight of the non-embossed section-forming portion 33 of the absorbent body 5. Specifically, the basis weights of each of the pair of embossed section-forming portions of the absorbent core 32 (32a, 32b) of the absorbent core 7 are lower than the basis weight of the non-embossed section-forming portion of the absorbent core 34 of the absorbent core 7. As shown in Fig. 10, this is because at the point before the pair of embossed sections 21 (21a, 21b) are formed, the absorbent body 5, and specifically the absorbent core 7, comprises high-basis-weight regions 35 and low-basis-weight regions 37 of lower basis weight than the basis weight of the high-basis-weight regions 35. The low-basis-weight regions 37 are formed so as to match with each of the pair of embossed section-forming portions 31 (31a, 31b) (specifically, each of the pair of embossed section-forming portions of the absorbent core 32 (32a, 32b)).

In the absorbent article 1 of the fourth embodiment, at the point before the pair of embossed sections 21 (21a, 21b) are formed, the absorbent body 5 (specifically the absorbent core 7) is formed so that each of the pair of embossed section-forming portions 31 (31a, 31b) (specifically, each of the pair of embossed section-forming portions of the absorbent core 32 (32a, 32b)) constitute low-basis-weight regions 37 and the non-embossed section-forming portion 33 (specifically, the non-embossed section-forming portion of the absorbent core 34) constitutes the high-basis-weight regions 35, and the same basis weight relationship is maintained even after the pair of embossed sections 21 (21a, 21b) have been formed. A lower basis weight of the absorbent body 5 allows the color of the colored layer 15 to penetrate more easily, and therefore from the liquid-permeable sheet 3 side, the color of the colored layer 15 is more easily visible through each of the pair of embossed sections 21 (21a, 21b), while the color of the colored layer 15 is less easily visible through the non-embossed section 27.

Furthermore, in the embossed region 29, the mass ratio of pulp fibers in each of the embossed section-forming portions of the absorbent core 32 (32a, 32b) of the absorbent core 7 is higher than the mass ratio of the pulp fibers 9 of the non-embossed section-forming portion of the absorbent core 34 of the non-embossed section 27. This is because, as shown in Fig. 3, at the point before the pair of embossed sections 21 (21a, 21b) are formed, the mass ratio of the pulp fibers 9 in the low-basis-weight regions 37 corresponding to the pair of embossed section-forming portions 31 (31a, 31b), in the absorbent core 7, is higher than the mass ratio of the pulp fibers 9 in the high-basis-weight regions 35 corresponding to the non-embossed section-forming portion, so that it has the same pulp proportion relationship even after the pair of embossed sections 21 (21a, 21b) have been formed.

In the absorbent core 7, it will be common for the refractive index of the pulp fibers 9 and the refractive index of the polymer absorbent 11 to be different, and for each of the pair of embossed sections 21 (21a, 21b) in the form of a film, a higher mass ratio of the pulp fibers 9 will cause incident light directed to each of the embossed sections 21 (21a, 21b) to be less likely to be reflected at the interface between the pulp fibers 9 and the polymer absorbent 11, more easily reaching the colored layer 15, compared to when the mass ratio of the pulp fibers 9 is low, and the incident light will reach the colored layer 15, and subsequently reflected light will more easily penetrate the embossed sections 21 (21a, 21b) and reach the eyes of the wearer.

At the non-embossed section 27, the absorbent core 7 includes the pulp fibers 9 and the polymer absorbent 11 in a state that also includes air. In this condition, if the mass ratio of the pulp fibers 9 of the absorbent core 7 is lowered, i.e. if the mass ratio of the polymer absorbent 11 of the absorbent core 7 is increased, in the non-embossed section 27, then compared to when it is not, a greater amount of light will be reflected at the interface between the pulp fibers 9 and the polymer absorbent 11, the interface between the pulp fibers 9 and air, and the interface between the polymer absorbent 11 and air, while incident light directed onto the non-embossed section 27 will be unlikely to reach the colored layer 15, and reflected light that has been reflected from the colored layer 15 will be unlikely to pass through the non-embossed section 27 and reach the eyes of the wearer.

By thus making the mass ratio of the pulp fibers 9 in the embossed section-forming portions of the absorbent core 32 (32a, 32b) higher than the mass ratio of the pulp fibers 9 in the non-embossed section-forming portion of the absorbent core 34, in the embossed region 29, the color of the colored layer 15 is more easily visible through the pair of embossed sections 21 (21a, 21b) while the color of the colored layer 15 is less easily visible through the non-embossed section 27, from the liquid-permeable sheet 3 side.

In addition, in the embossed region 29, the thickness of the embossed section-forming portions 31 (31a, 31b) of the absorbent body 5 is smaller than the thickness of the non-embossed section-forming portion 33 of the absorbent body 5. Thus, at the pair of embossed sections 21 (21a, 21b), the distance between the skin-contacting surface of the liquid-permeable sheet 3 and the colored layer 15 is physically closer than in the non-embossed section 27, and the color of the colored layer 15 is more easily visible through the pair of embossed sections 21 (21a, 21b) while the color of the colored layer 15 is less easily visible through the non-embossed section 27, from the liquid-permeable sheet 3 side.

In particular, in the absorbent body 5 at the point before the pair of embossed sections 21 (21a, 21b) are formed, the thicknesses of the embossed section-forming portions 31 (31a, 31b) forming the pair of embossed sections 21 (21a, 21b) are smaller than the thickness of the non-embossed section-forming portion 33 forming the non-embossed section 27. Specifically, in the absorbent core 7, at the point before the pair of embossed sections 21 (21a, 21b) are formed, the thicknesses of the embossed section-forming portions of the absorbent core 32 (32a, 32b) forming the pair of embossed sections 21 (21a, 21b) are smaller than the thickness of the non-embossed section-forming portion of the absorbent core 34 forming the non-embossed section 27. Therefore, at the pair of embossed sections 21 (21a, 21b), the distance between the skin-contacting surface of the liquid-permeable sheet 3 and the colored layer 15 is physically closer than in the non-embossed section 27, and the color of the colored layer 15 is more easily visible through the pair of embossed sections 21 (21a, 21b) while the color of the colored layer 15 is less easily visible through the non-embossed section 27, from the liquid-permeable sheet 3 side.

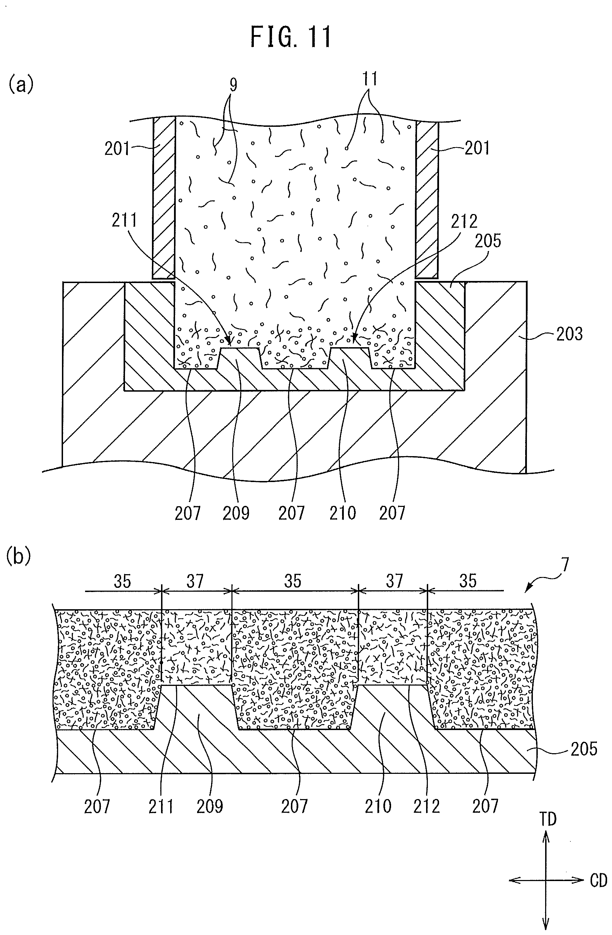

Fig. 11 is a diagram for illustration of a production example for the absorbent core 7 of the absorbent article 1 according to the first embodiment. Specifically, Fig. 11 (a) is a partial magnified end view schematically showing the state where pulp fibers 9 and a polymer absorbent 11 accumulate in a molding member 205 provided on the outer peripheral surface of a suction drum 203 that produces the absorbent core 7. Fig. 11(b) is a diagram illustrating an absorbent core 7 that has been accumulated on the molding member 205.

A conveying tube 201 that conveys the pulp fibers 9 and polymer absorbent 11, as the starting materials for the absorbent core 7, is connected to the suction drum 203. The molding member 205 situated on the outer peripheral surface of the suction drum 203 comprises a pair of protrusions 209, 210 having trapezoid cross-sections, and protruding from the base 207 and extending in the circumferential direction of the suction drum 203. Specifically, the pair of protrusions 209, 210 are each formed on the base 207 of the molding member 205, so that their shapes in the peripheral surface direction correspond to the shapes of the pair of embossed sections 21 (21a, 21b) in the lengthwise direction L, as shown in the first embodiment.

In the molding member 205, the base 207 is formed from a wire mesh with air permeability while the pair of protrusions 209, 210 are formed from metal plates without air permeability. In Fig. 11(a), the direction from back to front is the circumferential direction (rotational direction) of the suction drum 203. The suction drum 203 takes in air from the upper end to the lower end in Fig. 11(a).

As shown in Fig. 11(a), when the pulp fibers 9 and polymer absorbent 11 are supplied from the conveying tube 201 to the suction drum 203, the pulp fibers 9 and polymer absorbent 11 migrate lower as air is taken in by the suction drum 203. When the polymer absorbent 11 impacts with the respective top surfaces 211 and 212 of the protrusion 209 and protrusion 210, the polymer absorbent 11 flies upward, finally accumulating on the base 207. This occurs because the polymer absorbent 11 is generally particulate and has greater mass than the pulp fibers 9, so that it tends to fly off when it impacts with the end faces of the protrusion 209 and protrusion 210, while the fact that the protrusion 209 and protrusion 210 are formed of metal plates without air permeability such that air intake does not occur there, means that it is less likely to remain on the protrusion 209 and protrusion 210.

The pulp fibers 9, on the other hand, being lightweight and flexible, are less likely to fly off upon impact with the pair of protrusions 209, 210, and can thus accumulate on the top surfaces 211, 212. As mentioned above, since air intake by the suction drum 203 does not take place at the top surfaces 211, 212, the amount of pulp fibers 9 accumulating at the top surfaces 211, 212 is less than at the base 207.

As shown in Fig. 11(b), the low-basis-weight regions 37 of the absorbent core 7 are formed at the top surface 211 of the protrusion 209 and the top surface 212 of the protrusion 210, while the high-basis-weight regions 35 of the absorbent core 7 are formed on the base 207.

At the low-basis-weight regions 37, the polymer absorbent 11 is less likely to accumulate than at the high-basis-weight regions 35, and the accumulation rate of the pulp fibers 9 is more moderate, so that the low-basis-weight regions 37 have lower basis weight than the high-basis-weight regions 35 and the mass ratio of pulp fibers is increased with respect to the total mass of the pulp fibers 9 and polymer absorbent 11.

The basis weights and mass ratio of pulp fibers in the high-basis-weight regions 35 and low-basis-weight regions 37 can be adjusted by controlling the amounts of pulp fibers 9 and polymer absorbent 11 supplied to the suction drum 203, and their mass ratio, the level of air intake per unit time at the suction drum 203, and the air permeability of the pair of protrusions 209, 210 and the air permeability of the base.

In the absorbent article of the present disclosure, the materials for the liquid-permeable sheet, absorbent body (core wrap and absorbent core), and liquid-impermeable sheet may be ones commonly used in the technical field, without any particular restrictions.

In particular, the materials of the liquid-permeable sheet and core wrap may be selected from among fabrics (for example, nonwoven fabrics, woven fabrics and knitted fabrics) and sheets, such as porous resin sheets, with fabrics being preferred. This is from the viewpoint of helping to ensure light permeability at the embossed sections, and helping to ensure light impermeability at the non-embossed sections.

When the liquid-permeable sheet in the absorbent article of the present disclosure includes a pigment (for example, a white pigment, such as titanium oxide), the liquid-permeable sheet includes the pigment at a concentration of preferably 0.1 to 20.0 mass%, more preferably 0.5 to 10.0 mass% and even more preferably 1.0 to 5.0 mass%. This is from the viewpoint of helping to ensure light permeability at the embossed sections, and helping to ensure light impermeability at the non-embossed sections.

Pigments, such as titanium oxide have higher refractive indexes than thermoplastic resins which are commonly used as liquid-permeable sheet materials, and increasing the amount of titanium oxide in the liquid-permeable sheet tends to aid reflection of light at the interface between the thermoplastic resin and the titanium oxide, thus lowering the light permeability.

The liquid-permeable sheet in the absorbent article of the present disclosure has a basis weight of preferably 10 to 100 g/m$^2$, more preferably 12 to 70 g/m$^2$ and even more preferably 15 to 35 g/m$^2$. This is from the viewpoint of helping to ensure light permeability at the embossed sections, and helping to ensure light impermeability at the non-embossed sections.

Throughout the present specification, the basis weight is calculated by cutting out a sample to a size of 3 cm × 3 cm, for example, for an area of 100 cm$^2$, and measuring its mass.

In the absorbent article of the present disclosure, the absorbent body has an average basis weight of preferably 50 to 1,000 g/m$^2$, more preferably 75 to 750 g/m$^2$ and even more preferably 100 to 500 g/m$^2$. This is from the viewpoint of helping to ensure light permeability at the embossed sections, and helping to ensure light impermeability at the non-embossed section.

In the absorbent article of the present disclosure, the embossed region that includes the pair of embossed sections and the non-embossed section situated between them may be disposed at any desired location of the absorbent article, and for example, when the absorbent article is to be used by anchoring onto clothing, such as in the case of a sanitary napkin, panty liner or urine-absorbing pad, the embossed region is preferably disposed in a region including the excretory opening contact region (for example, the vaginal opening-contacting region or the urine orifice-contacting region), and more preferably it is disposed in the excretory opening contact region. The embossed region may also be disposed inside the excretory opening contact region. This is because matching the excretory opening contact region of the absorbent article with the location of the excretory opening of the wearer is preferred from the viewpoint of the absorption property.

The absorbent article of the present disclosure may also comprise a plurality of embossed regions, and separate embossed regions may also be formed inside some of the embossed regions. By having separate embossed regions formed in the embossed region, visibility of the embossed regions will be increased and the wearer will be able to more easily achieve positioning.

When the absorbent article of the disclosure comprises a plurality of embossed regions, the preferred properties for each of the plurality of embossed regions is the same as described above.

When the absorbent article is to be used by anchoring onto clothing and the embossed region is disposed in the excretory opening contact region, preferably the non-embossed section is disposed in the excretory opening contact region and the pair of embossed sections continuously or intermittently surround the outer edges of the excretory opening contact region. This is from the viewpoint of serving as a positioning mark.

For the purpose of the present specification, the pair of embossed sections in the embossed region are sufficient if they sandwich the non-embossed section between them, and each of the pair of embossed sections may have mutually differing shapes. From the viewpoint of easier positioning, each of the pair of embossed sections described in the present specification preferably have shapes with symmetry (point symmetry or line symmetry) around the non-embossed section as the center.

Also, each of the pair of embossed sections may be connected together to form a single connected embossed section. The connected embossed section may extend continuously or intermittently.

When the absorbent article of the present disclosure is not to be used by anchoring to clothing, such as in the case of a disposable diaper, the embossed region is preferably provided with the excretory opening contact region, and especially the crotch region, as the center. This is from the viewpoint of the absorption property, as well as usage of the embossed sections of the embossed region as folding guides.

The embossed sections forming the embossed region in the absorbent article of the present disclosure have at least the absorbent body embossed, but the colored layer is not embossed. In other words, the constituent element of the embossed sections in the thickness direction is the absorbent body, the absorbent body being the lower bound constituent element. This is from the viewpoint of the effect of the present disclosure.

The embossed sections in the absorbent article of the present disclosure may have only the absorbent body embossed. In other words, the embossed sections may have only the absorbent body as the constituent element, with the absorbent body as both the upper bound and lower bound constituent elements. This is because forming the embossed sections in the absorbent body will allow the colored layer to be visible from the liquid-permeable sheet side of the absorbent article.

The embossed sections in the absorbent article of the present disclosure preferably are embossed at the layer reaching from the liquid-permeable sheet to the absorbent body. In other words, the embossed sections preferably have the layer from the liquid-permeable sheet to the absorbent body as the constituent element, with the liquid-permeable sheet as the upper bound of the constituent element and the absorbent body as the lower bound constituent element. This is from the viewpoint of visibility of the colored layer from the liquid-permeable sheet side of the absorbent article.

In the absorbent article of the present disclosure, the embossed sections forming the embossed region may be formed by a method commonly employed in the technical field, such as an embossing method in which an object is embossed with a pair of embossing rolls. From the viewpoint of increasing light permeability at the embossed sections, is it preferred to increase the embossing temperature, as the temperature to which the object is subjected in the embossing method. For example, when the embossed sections forming the embossed region are to be formed in the liquid-permeable sheet and absorbent body, and the liquid-permeable sheet is made of a nonwoven fabric of thermoplastic resin fibers having a core-sheath structure, the embossing temperature may be set to a higher temperature than the melting point of the sheath component of the thermoplastic resin fibers.

In the absorbent article of the present disclosure, the thicknesses of the embossed section-forming portions of the absorbent body are smaller than the thickness of the non-embossed section-forming portion of the absorbent body, and preferably a thickness of 0.1 to 30% and more preferably 0.5 to 10% of the thickness of the non-embossed section-forming portion of the absorbent body. This is from the viewpoint of helping to ensure light permeability at the embossed sections, and helping to ensure light impermeability at the non-embossed sections.

The thickness can be measured by cutting the absorbent body that has been frozen with liquid nitrogen or the like and taking an image of the cut surface with an electron microscope and performing measurement from the image.

The embossed section-forming portions of the absorbent body in the absorbent article of the present disclosure preferably have lower basis weight than the non-embossed section-forming portion of the absorbent body. That is, preferably the embossed section-forming portions of the absorbent body constitute the low-basis-weight regions while the non-embossed section-forming portions of the absorbent body constitute the high-basis-weight regions. The embossed section-forming portions have a basis weight of preferably 1 to 70 mass% and more preferably 5 to 50 mass% of the average basis weight of the absorbent body. This is from the viewpoint of helping to ensure light permeability at the embossed sections, and helping to ensure light impermeability at the non-embossed sections.

The basis weight of the embossed section-forming portions is calculated by cutting out an embossed section-forming portion from the absorbent body and measuring its area and mass.

When the absorbent body in the absorbent article of the present disclosure comprises an absorbent core that includes pulp fibers and a polymer absorbent, the absorbent article includes the pulp fibers and polymer absorbent in the absorbent core at proportions of preferably 30 to 99 mass% and 1 to 70 mass%, more preferably 45 to 95 mass% and 5 to 55 mass%, and even more preferably 60 to 90 mass% and 10 to 40 mass%, based on 100% as the total mass.

When the absorbent body in the absorbent article of the present disclosure comprises an absorbent core including pulp fibers and a polymer absorbent, the mass ratio of pulp fibers of the embossed section-forming portions of the absorbent core is preferably higher, more preferably at least 5% higher, more preferably at least 10% higher and even more preferably at least 20% higher than the mass ratio of pulp fibers of the non-embossed section-forming portion of the absorbent core in the non-embossed section. This is from the viewpoint of helping to ensure light permeability at the embossed sections, and helping to ensure light impermeability at the non-embossed sections.

The mass ratio of pulp fibers is the ratio of the mass of pulp fibers in the absorbent core with respect to the total mass of the components forming the absorbent core.

The mass ratio of pulp fibers in the embossed section-forming portions of the absorbent core is calculated by cutting out an embossed section-forming portion of the absorbent core, separating the pulp fibers from the other components, and measuring the mass of the pulp fibers and the total mass of all of the components forming the absorbent core. The mass ratio of pulp fibers in the non-embossed section-forming portion of the absorbent core is measured in the same manner.

The non-embossed section in the embossed region of the absorbent article of the present disclosure does not have the absorbent body and colored layer embossed, although the liquid-permeable sheet may be embossed in the non-embossed section. A spunbonded nonwoven fabric is an example of such a liquid-permeable sheet.

The absorbent article of the disclosure may include a second absorbent body between the absorbent body and the liquid-impermeable sheet. The sheet composing the skin-contacting surface of the second absorbent body preferably constitutes the colored layer. This is from the viewpoint of allowing a color shade to be selected that easily serves as a mark for positioning of the absorbent article from the liquid-permeable sheet side of the absorbent article, regardless of the color shade formed on the clothing-contacting surface of the liquid-impermeable sheet.

The colored layer in the absorbent article of the disclosure is a separate constituent element from the liquid-impermeable sheet. If the colored layer in the absorbent article of the disclosure is separate from the liquid-impermeable sheet, the color of the colored layer may be selected so as to serve as a positioning mark for the absorbent article, regardless of the color shade formed on the clothing-contacting surface of the liquid-impermeable sheet.

The colored layer in the absorbent article of the disclosure is not particularly restricted so long as it is a colored layer, and examples include fabrics, such as nonwoven fabrics, woven fabrics and textiles, and sheets, such as resin sheets.

The colored layer may be purchased, or it may be formed by coating or printing a colored material onto a white nonwoven fabric or a transparent or semi-transparent resin film.

The colored layer in the absorbent article of the disclosure may be disposed in a region overlapping with the embossed regions in the thickness direction of the absorbent article. This is from the viewpoint of the effect of the present disclosure. The colored layer may also be situated in a region overlapping with the absorbent body in the thickness direction, and it may be disposed extending beyond the absorbent body in the lengthwise direction and widthwise direction of the absorbent article.

The colored layer in the absorbent article of the disclosure has a different color from the liquid-permeable sheet. The colored layer has a color difference of preferably 3.0 or greater, more preferably 5.0 or greater and even more preferably 8.0 or greater with the liquid-permeable sheet. This will tend to make the color of the colored layer more easily visible to the wearer through the embossed sections in the embossed region, from the liquid-permeable sheet side.

The colored layer also has a color difference of preferably no greater than 50, more preferably no greater than 40 and even more preferably no greater than 30 with the liquid-permeable sheet. This will tend to make the color of the colored layer less easily visible to the wearer through the non-embossed section in the embossed region, from the liquid-permeable sheet side.

The color difference is measured in the following manner.

(1) A Chroma Meter CR-300 by Konica Minolta Holdings, Inc. is prepared in a thermostatic chamber at 20°C, and the liquid-permeable sheet and colored layer to be measured, and qualitative filter paper No.2 φ55 by Advantec Co. (JIS P 3801), are prepared and allowed to stand for 1 hour.

(2) The liquid-permeable sheet to be measured is set on the qualitative filter paper, with the skin-contacting surface facing upward, and the coordinates ($L*_0$, a*0, $b*_0$) of the skin-contacting surface according to the CIE L*a*b* color system are measured using the color difference meter.

(3) The colored layer is also set on the qualitative filter paper, with the skin side surface facing upward, and the coordinates (L*1, a*1, b*1) according to the CIE L*a*b* color system are measured using the color difference meter.

(4) The color difference ($\Delta$E) is calculated by the following formula.

$$\Delta E = \{(L^*_1 - L^*_0)^2 + (a^*_1 - a^*_0)^2 + (b^*_1 - b^*_0)^2\}^{0.5}$$

(5) The measurement is conducted 5 times, and the average value is recorded.

At the embossed sections of the embossed region, the liquid-permeable sheet preferably has a lighter color, and more preferably it has a white color, from the viewpoint of visibility of the color of the colored layer from the liquid-permeable sheet side of the absorbent article. More specifically, from this viewpoint, the liquid-permeable sheet has, on the skin-contacting surface, an $L*_0$ value of preferably 80 or greater, more preferably 85 or greater, even more preferably 90 or greater and yet more preferably 95 or greater, among the coordinates (L*0, a*0, b*0) in the CIE L*a*b* color system.

When the absorbent article of the disclosure includes a first absorbent body and a second absorbent body, with the skin surface of the second absorbent body forming the colored layer and the first absorbent body having an additional colored layer (for example, when the core wrap of the first absorbent body closer to the liquid-impermeable sheet is colored and constitutes the additional colored layer), preferably the colored layer of the second absorbent body and the additional colored layer of the first absorbent body have similar color tones, and more preferably the colored layer of the second absorbent body and the additional colored layer of the first absorbent body have similar color tones while the chroma of the colored layer of the second absorbent body is higher than the chroma of the additional colored layer of the first absorbent body. This will increase the brilliance of the embossed sections in the embossed region, and improve the visibility of the embossed region.

The colored layer may also include a functional component. Examples for functional components include components selected from the group consisting of aromatic components, cooling components, warming components, deodorant components, antimicrobial components, skin care components and any desired combinations of the foregoing.

The aromatic components are not particularly restricted so long as they are used as aromatics in the technical field, and examples include highly volatile aromatics with boiling points of no higher than about 250°C, and moderately volatile aromatics with boiling points of about 250°C to about 300°C.

The cooling components include those known as cooling materials in the technical field, and examples include components that act on receptor-activated channels in skin nerves (TRPM8), such as menthol (for example, 1-menthol) and its derivatives, methyl salicylate, camphor, and essential oils derived from plants (such as mint or eucalyptus). The cooling components also include components that lower the surrounding temperature by heat of vaporization, including alcohols such as methanol and ethanol.

Examples of the warming components include capsicoside, capsaicin, capsaicinoids (dihydrocapsaicin, nordihydrocapsaicin, homodihydrocapsaicin, homocapsaicin, nonivamide and the like), capsanthin, benzyl nicotinate, β-butoxyethyl nicotinate, N-acylvanillamide, nonanoic acid vanillylamide, polyhydric alcohols, red pepper powder, red pepper tincture, red pepper extract, nonanoic acid vanillyl ether, vanillyl alcohol alkyl ether derivatives (for example, vanillyl ethyl ether, vanillyl butyl ether, vanillyl pentyl ether and vanillyl hexyl ether), isovanillyl alcohol alkyl ethers, ethyl vanillyl alcohol alkyl ethers, veratryl alcohol derivatives, substituted benzyl alcohol derivatives, substituted benzyl alcohol alkyl ethers, vanillin propyleneglycol acetal, ethylvanillin propyleneglycol acetal, ginger extract, ginger oil, gingerol, zingerone, and any desired combinations of the foregoing.

The deodorant components may be components known as deodorants in the technical field.

Examples of the skin care components include anti-inflammatory components, antipruritic components, anti-eruption components and moisturizing components, examples including menthol and methyl salicylate.

Examples of the antimicrobial components include quaternary ammonium salts, guanidine-based antimicrobial agents (for example, chlorhexidine gluconate and chlorhexidine hydrochloride), biguanide-based antimicrobial agents, metal ion-supporting agents, hexetidine and metallonidazole.

REFERENCE SIGNS LIST

1 Absorbent article

3 Liquid-permeable sheet
5 Absorbent body
7 Absorbent core
9 Pulp fibers
11 Polymer absorbent
13 Core wrap
15 Colored layer
17 Liquid-impermeable sheet
19 Anchoring part
21, 22, 23, 24, 25 Embossed section
27 Non-embossed section
29 Embossed region
31 Embossed section-forming portion
32 Embossed section-forming portion of absorbent core
33 Non-embossed section-forming portion
34 Non-embossed section-forming portion of absorbent core
35 High-basis-weight region
37 Low-basis-weight region
51 First absorbent body
53 Second absorbent body
55 Polymer absorbent layer
57 Second upper core wrap
59 Second lower core wrap
61 Side edge
63, 65 Notch section
71 First non-embossed section
73 First embossed section
75 First embossed region
77 Second embossed region
79, 81, 83 Second non-embossed section
85, 87, 89 Second embossed section
91 Absorbent core second embossed section-forming portion
93 Absorbent core second non-embossed section-forming portion

**Claims**

1. An absorbent article comprising a liquid-permeable sheet (3), an absorbent body (5), a colored layer (15) and a liquid-impermeable sheet (17), in that order, and having a lengthwise direction (L), a widthwise direction (W) and a thickness direction (T), wherein

   the absorbent article (1) comprises an embossed region (29) including a pair of embossed sections (21, 24) and a non-embossed section (27) situated between them,
   each of the pair of embossed sections (21, 24) has the absorbent body (5) embossed but does not have the colored layer (15) embossed,
   the non-embossed section (27) does not have the absorbent body (5) and colored layer (15) embossed, and
   in the embossed region (21, 24), thicknesses of embossed section-forming portions (31) of the absorbent body (5) forming each of the pair of embossed sections (21, 24) are smaller than a thickness of a non-embossed section-forming portion (33) of the absorbent body (5) forming the non-embossed section (27), **characterised in that** in the embossed region (29), a basis weight of the embossed section-forming portions (31) of the absorbent body (5) is lower than a basis weight of the non-embossed section-forming portion (33) of the absorbent body (5).

2. The absorbent article according to claim 1, wherein the absorbent body (1) comprises an absorbent core (7) including pulp fibers and a polymer absorbent, and in the absorbent core (7), a mass ratio of the pulp fibers of the embossed section-forming portions (31) of the absorbent core (7) forming each of the pair of embossed sections (21, 24) is higher than a mass ratio of the pulp fibers of the non-embossed section-forming portion (33) of the absorbent core (7) forming the non-embossed section (27).

3. The absorbent article according to any one of claims 1 to 2, wherein each of the pair of embossed sections (21, 24) has the liquid-permeable sheet (3) further embossed in the thickness direction (T).

4. The absorbent article according to any one of claims 1 to 3, wherein the embossed region (29) is disposed in a region including a excretory opening contact region of the absorbent article (1).

5. The absorbent article according to claim 4, wherein the absorbent article (1) has an anchoring part (19) in the liquid-impermeable sheet (3) for anchoring of the absorbent article to clothing, and the pair of embossed sections (29) extend in the lengthwise direction at both outer sides of the non-embossed section (27) in the widthwise direction (W).

6. The absorbent article according to claim 5, wherein the non-embossed section (27) is disposed in the excretory opening contact region, and the pair of embossed sections (29) continuously or intermittently surround an outer edge of the excretory opening contact region.

7. The absorbent article according to claim 6, wherein the absorbent article (1) comprises, inside of the embossed region (29), a second embossed region (77) that includes a pair of second embossed sections (85) and a second non-embossed section (79) situated between them, each of the pair of second embossed sections (85) has the liquid-permeable sheet (3) and absorbent body (5) embossed but does not have the colored layer (15) embossed, and the second non-embossed section (79) does not have the absorbent body (5) and colored layer (15) embossed.

8. The absorbent article according to any one of claims 1 to 4, wherein the absorbent article (1) is a disposable diaper, and each of the pair of embossed sections is a folding guide extending in the lengthwise direction.

9. The absorbent article according to any one of claims 1 to 8, wherein the absorbent article (1) comprises a second absorbent body (53) between the absorbent body (5) and the liquid-impermeable sheet (17) in the thickness direction (T), with the colored layer (15) forming a skin surface of the second absorbent body (53).

10. The absorbent article according to any one of claims 1 to 9, wherein the colored layer (15) further includes a functional component selected from the group consisting of aromatic components, cooling components, warming components, deodorant components, antimicrobial components, skin care components and any combinations of the foregoing.


**Patentansprüche**

1. Absorbierender Artikel mit einer flüssigkeitsdurchlässigen Lage (3), einem absorbierenden Körper (5), einer gefärbten Schicht (15) und einer flüssigkeitsundurchlässigen Lage (17) in dieser Reihenfolge und mit einer Längsrichtung (L), einer Breitenrichtung (W) und einer Dickenrichtung (T), wobei

der absorbierende Artikel (1) einen geprägten Bereich (29) mit einem Paar von geprägten Abschnitten (21, 24) und einem dazwischen angeordneten nicht geprägten Abschnitt (27) aufweist,
bei jedem der beiden geprägten Abschnitte (21, 24) der absorbierende Körper (5) geprägt ist, aber nicht die farbige Schicht (15) geprägt ist,
der nicht geprägte Abschnitt (27) keinen geprägten Absorptionskörper (5) und keine geprägte Farbschicht (15) aufweist, und
in dem geprägten Bereich (21, 24) die Dicken von geprägten abschnittsausbildenden Abschnitten (31) des absorbierenden Körpers (5), die jeden des Paars von geprägten Abschnitten (21, 24) ausbilden, kleiner sind als eine Dicke eines nicht geprägten abschnittsausbildenden Abschnitts (33) des absorbierenden Körpers (5), der den nicht geprägten Abschnitt (27) ausbildet, **dadurch gekennzeichnet, dass** in dem geprägten Bereich (29) ein Flächengewicht der geprägten abschnittsausbildenden Abschnitte (31) des Absorptionskörpers (5) niedriger ist als ein Flächengewicht des nicht geprägten, abschnittsausbildenden Abschnitts (33) des Absorptionskörpers (5).

2. Absorbierender Artikel gemäß Anspruch 1, wobei der absorbierende Körper (1) einen absorbierenden Kern (7) aufweist, der Zellstofffasern und ein polymerisches Absorptionsmittel umfasst, und in dem absorbierenden Kern (7) ein Massenverhältnis der Zellstofffasern der einen geprägten Abschnitt ausbildenden Anschnitte (31) des absorbierenden Kerns (7), die jeden des Paars von geprägten Abschnitten (21, 24) ausbilden, höher ist als ein Massenverhältnis der Zellstofffasern des einen nicht geprägten Abschnitt ausbildenden Abschnitts (33) des absorbierenden Kerns (7), der den nicht geprägten Abschnitt (27) ausbildet.

3. Absorbierender Artikel gemäß einem der Ansprüche 1 bis 2, wobei jeder der beiden geprägten Abschnitte (21, 24) die flüssigkeitsdurchlässige Folie (3) aufweist, die in der Dickenrichtung (T) weiter geprägt ist.

4. Absorbierender Artikel gemäß einem der Ansprüche 1 bis 3, wobei der geprägte Bereich (29) in einem Bereich angeordnet ist, der einen Kontaktbereich der Ausscheidungsöffnung des absorbierenden Artikels (1) umfasst.

5. Absorbierender Artikel gemäß Anspruch 4, wobei der absorbierende Artikel (1) ein Verankerungsteil (19) in der flüssigkeitsundurchlässigen Lage (3) zur Verankerung des absorbierenden Artikels an der Kleidung aufweist, und das Paar von geprägten Abschnitten (29) sich in Längsrichtung an beiden Außenseiten des nicht geprägten Abschnitts (27) in Breitenrichtung (W) erstreckt.

6. Absorbierender Artikel gemäß Anspruch 5, wobei der nicht geprägte Abschnitt (27) in dem Kontaktbereich der Ausscheidungsöffnung angeordnet ist und das Paar von geprägten Abschnitten (29) kontinuierlich oder intermittierend eine Außenkante des Kontaktbereichs der Ausscheidungsöffnung umgibt.

7. Absorbierender Artikel gemäß Anspruch 6, wobei der absorbierende Artikel (1) auf der Innenseite des geprägten Bereichs (29) einen zweiten geprägten Bereich (77) aufweist, der ein Paar von zweiten geprägten Abschnitten (85) und einen zweiten nicht geprägten Abschnitt (79) aufweist, der zwischen diesen angeordnet ist, wobei jeder des Paares von zweiten geprägten Abschnitten (85) die flüssigkeitsdurchlässige Folie (3) und den absorbierenden Körper (5) geprägt aufweist, aber nicht die farbige Schicht (15) geprägt aufweist, und der zweite nicht geprägte Abschnitt (79) den absorbierenden Körper (5) und die farbige Schicht (15) nicht geprägt aufweist.

8. Absorbierender Artikel gemäß einem der Ansprüche 1 bis 4, wobei der absorbierende Artikel (1) eine Wegwerfwindel ist und jeder der beiden geprägten Abschnitte eine sich in Längsrichtung erstreckende Faltführung ist.

9. Absorbierender Artikel gemäß einem der Ansprüche 1 bis 8, wobei der absorbierende Artikel (1) einen zweiten absorbierenden Körper (53) zwischen dem absorbierenden Körper (5) und der flüssigkeitsundurchlässigen Lage (17) in der Dickenrichtung (T) aufweist, wobei die farbige Schicht (15) eine Hautoberfläche des zweiten absorbierenden Körpers (53) ausbildet.

10. Absorbierender Artikel gemäß einem der Ansprüche 1 bis 9, wobei die farbige Schicht (15) ferner eine funktionelle Komponente umfasst, die aus der Gruppe bestehend aus aromatischen Komponenten, kühlenden Komponenten, wärmenden Komponenten, deodorierenden Komponenten, antimikrobiellen Komponenten, Hautpflegekomponenten und beliebigen Kombinationen der vorgenannten ausgewählt ist.

## Revendications

1. Article absorbant comprenant une feuille perméable aux liquides (3), un corps absorbant (5), une couche colorée (15) et une feuille imperméable aux liquides (17), dans cet ordre, et ayant une direction dans le sens de la longueur (L), une direction dans le sens de la largeur (W) et une direction dans le sens de l'épaisseur (T), dans lequel

   l'article absorbant (1) comprend une région gaufrée (29) comportant une paire de sections gaufrées (21, 24) et une section non gaufrée (27) situées entre elles,
   chacune de la paire de sections gaufrées (21, 24) présente le corps absorbant (5) gaufré mais ne présente pas la couche colorée (15) gaufrée,
   la section non gaufrée (27) ne présente pas le corps absorbant (5) ni la couche colorée (15) gaufrée, et
   dans la région gaufrée (21, 24), les épaisseurs de parties de formation de section gaufrée (31) du corps absorbant (5) formant chacune de la paire de sections gaufrées (21, 24) sont plus petites qu'une épaisseur d'une partie de formation de section non gaufrée (33) du corps absorbant (5) formant la section non gaufrée (27), **caractérisé en ce que**
   dans la région gaufrée (29), un poids de base des parties de formation de section gaufrée (31) du corps absorbant (5) est inférieur à un poids de base de la partie de formation de section non gaufrée (33) du corps absorbant (5),

2. Article absorbant selon la revendication 1, dans lequel le corps absorbant (1) comprend un noyau absorbant (7) comportant des fibres de pâte et un absorbant polymère, et dans le noyau absorbant (7), un rapport de masse des fibres de pâte des parties de formation de section gaufrée (31) du noyau absorbant (7) formant chacune de la paire de sections gaufrées (21, 24) est supérieur à un rapport de masse des fibres de pâte de la partie de formation de section non gaufrée (33) du noyau absorbant (7) formant la section non gaufrée (27).

3. Article absorbant selon l'une quelconque des revendications 1 et 2, dans lequel chacune de la paire de sections gaufrées (21, 24) présente la feuille perméable aux liquides (3) encore gaufrée dans la direction dans le sens de l'épaisseur (T).

4. Article absorbant selon l'une quelconque des revendications 1 à 3, dans lequel la région gaufrée (29) est disposée dans une région comportant une région de contact d'ouverture d'excrétion de l'article absorbant (1).

5. Article absorbant selon la revendication 4, dans lequel l'article absorbant (1) présente une partie d'ancrage (19) dans la feuille imperméable aux liquides (3) pour ancrer l'article absorbant aux vêtements, et la paire de sections gaufrées (29) s'étendent dans la direction dans le sens de la longueur au niveau des deux côtés extérieurs de la section non gaufrée (27) dans la direction dans le sens de la largeur (W).

6. Article absorbant selon la revendication 5, dans lequel la section non gaufrée (27) est disposée dans la région de contact d'ouverture d'excrétion, et la paire de sections gaufrées (29) entourent de façon continue ou intermittente un bord extérieur de la région de contact d'ouverture d'excrétion.

7. Article absorbant selon la revendication 6, dans lequel l'article absorbant (1) comprend, à l'intérieur de la région gaufrée (29), une seconde région gaufrée (77) qui comporte une paire de secondes sections gaufrées (85) et une seconde section non gaufrée (79) située entre elles, chacune de la paire de secondes sections gaufrées (85) présente la feuille perméable aux liquides (3) et le corps absorbant (5) gaufré mais ne présente pas la couche colorée (15) gaufrée, et la seconde section non gaufrée (79) ne présente pas le corps absorbant (5) et la couche colorée (15) gaufrée.

8. Article absorbant selon l'une quelconque des revendications 1 à 4, dans lequel l'article absorbant (1) est une couche jetable, et chacune de la paire de sections gaufrées est un guide de pliage s'étendant dans la direction dans le sens de la longueur.

9. Article absorbant selon l'une quelconque des revendications 1 à 8, dans lequel l'article absorbant (1) comprend un second corps absorbant (53) entre le corps absorbant (5) et la feuille imperméable aux liquides (17) dans la direction dans le sens de l'épaisseur (T), la couche colorée (15) formant une surface cutanée du second corps absorbant (53).

10. Article absorbant selon l'une quelconque des revendications 1 à 9, dans lequel la couche colorée (15) comporte en outre un composant fonctionnel choisi dans le groupe constitué de composants aromatiques, de composants de refroidissement, de composants chauffants, de composants déodorants, de composants antimicrobiens, de composants de soin de la peau et de toutes les combinaisons de ce qui précède.

FIG. 1

EP 3 520 752 B1

FIG. 2

EP 3 520 752 B1

# FIG. 3

FIG. 4

# FIG. 5

EP 3 520 752 B1

FIG. 6

# FIG. 7

# FIG. 8

FIG. 9

# FIG. 10

EP 3 520 752 B1

# FIG. 11

(a)

(b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013075082 A

- US 2009012491 A1